(19) **Europäisches Patentamt / European Patent Office / Office européen des brevets**

(11) **EP 4 530 342 A1**

(12) **EUROPEAN PATENT APPLICATION**
published in accordance with Art. 153(4) EPC

(43) Date of publication:
02.04.2025 Bulletin 2025/14

(21) Application number: 23810585.2

(22) Date of filing: 03.03.2023

(51) International Patent Classification (IPC):
*C12N 1/18* (2006.01)          *A21D 8/04* (2006.01)
*C12R 1/865* (2006.01)

(52) Cooperative Patent Classification (CPC):
**A21D 8/04; C12N 1/18;** C12R 2001/865;
Y02E 50/10

(86) International application number:
**PCT/CN2023/079577**

(87) International publication number:
**WO 2023/226509 (30.11.2023 Gazette 2023/48)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 27.05.2022 CN 202210592367

(71) Applicant: Angel Yeast Co., Ltd
Yichang, Hubei 443003 (CN)

(72) Inventors:
• **SUN, Yafang**
Yichang, Hubei 443003 (CN)

• **XIAO, Minghua**
Yichang, Hubei 443003 (CN)
• **CHEN, Liting**
Yichang, Hubei 443003 (CN)
• **GUO, Tianfen**
Yichang, Hubei 443003 (CN)
• **WANG, Long**
Yichang, Hubei 443003 (CN)
• **KUANG, Jinbao**
Yichang, Hubei 443003 (CN)

(74) Representative: **karo IP**
Patentanwälte PartG mbB
Steinstraße 16-18
40212 Düsseldorf (DE)

(54) **YEAST STRAIN FOR FAST-FERMENTING SUGAR-FREE DOUGH AND USE THEREOF**

(57) The present invention provides a yeast strain for fast-fermenting sugar-free dough and uses thereof. The Saccharomyces cerevisiae AMCC31248 strain (Saccharomyces cerevisiae AMCC31248) provided by the present invention is deposited at the China Center for Type Culture Collection (CCTCC) with the deposit number CCTCC NO: M 20211686. The strain of Saccharomyces cerevisiae AMCC31248 strain provided by the present invention has good fermentation performance in sugar-free dough and can ferment sugar-free dough rapidly.

FIG. 1

EP 4 530 342 A1

## Description

## Technical Field

The present invention relates to the field of microorganisms and in particular to a yeast strain for fast-fermenting sugar-free dough and use thereof.

## Background Art

From the discovery of commercial baker's yeast to the formal formation of the yeast production process, to the development stage of active dry yeast nowadays, the variety of yeast products are increasingly diversified, and product quality are increasingly improved, and the yeast products can be used in the production of different flour food. As a kind of traditional staple food, fermented wheaten food occupies an important position in the people's dietary structure. According to statistics, about half of the population in China now takes fermented wheaten food as its staple food, and most of them are steamed bread, steamed stuffed buns and other fermented wheaten food fermented from dough without sugar. Sugar-free or low-sugared flour food is preferred by consumers from a health standpoint.

In flour food making, the dough fermentation process always plays a crucial role, not only affecting the flour product softness, taste, nutritional value, etc. but also determining the speed of the dough leavening. The stronger the yeast's fermentation ability, the faster the dough will rise, thereby shortening the fermentation cycle, speeding up the flour food-making process, and improving industrial production efficiency. Therefore, it is of great practical significance to develop Saccharomyces cerevisiae strains with good fermentation performance in sugar-free dough.

## Summary of the Invention

During the flour food making process, the temperature of the environment is often too high, which causes the yeast to start too fast during kneading dough and causes the quality of the product to decline. In order to reduce the temperature of the dough, low-temperature water is usually added to reduce the temperature, but the yeast cells will enter into a shock state when they encounter a low-temperature environment, thus affecting the normal growth of yeast cells. There is therefore an urgent need for cold osmotic shock resistance of yeast.

Therefore, in view of the problems of low efficiency of fermenting sugar-free dough with Saccharomyces cerevisiae and poor cold osmotic shock resistance in the prior art, the present invention provides a yeast strain for fast-fermenting sugar-free dough with cold osmotic shock resistance.

In a first aspect, the present invention provides a Saccharomyces cerevisiae strain comprising: Saccharomyces cerevisiae AMCC31248 strain (Saccharomyces cerevisiae AMCC31248) deposited at the China Center for Type Culture Collection (CCTCC) with the deposit number CCTCC NO: M 20211686.

In a second aspect, the present invention provides a fermentative preparation method for a Saccharomyces cerevisiae microbial agent, the process comprising the following steps: culturing the Saccharomyces cerevisiae strain.

Preferably, the preparation method comprises the following steps:

(1) amplifying and culturing the Saccharomyces cerevisiae strain;
(2) adding the product obtained in step (1) to a liquid culture medium, and fermenting and culturing at 26-32°C.

In a third aspect, the present invention provides a microbial agent containing the strain of Saccharomyces cerevisiae AMCC31248 strain (Saccharomyces cerevisiae AMCC31248). Preferably, the microbial agent is obtained by the fermentative preparation method.

In a fourth aspect, the present invention also provides the use of the Saccharomyces cerevisiae strain or the microbial agent in fermentation.

In a fifth aspect, the present invention also provides the use of the microbial agent of the Saccharomyces cerevisiae strain in a dough.

In a sixth aspect, the present invention provides a dough containing the Saccharomyces cerevisiae strain or the microbial agent.

Preferably, the dough contains flour and the Saccharomyces cerevisiae strain in a mass ratio of 100:0.5-5.

In a seventh aspect, the present invention also provides a preparation method for a dough comprising the following steps: kneading the dough with water at 0-35°C.

The water at 0°C in the present invention may be ice at 0°C or a mixture of ice and water at 0°C or liquid water at 0°C.

In the preparation of the dough according to the present invention, the water, the flour, and the Saccharomyces cerevisiae may be added in any order, such as mixing the flour and the Saccharomyces cerevisiae and then adding water at 0-35°C for kneading dough, adding the flour to water at 0-35°C and then adding the Saccharomyces cerevisiae, or adding the Saccharomyces cerevisiae to water at 0-35°C and then adding the flour.

In an eighth aspect, the present invention also provides a flour product obtainable by the dough preparation method.

Preferably, the flour product is a steamed bread, a steamed stuffed bun, a bread, a biscuit, a noodle, a pan-fried dumpling, and the like.

The present invention provides Saccharomyces cerevisiae AMCC31248 strain (Saccharomyces cerevisiae AMCC31248) provided by the present invention has good fermentation performance in sugar-free dough, can ferment sugar-free dough rapidly, and has good cold osmotic shock resistance.

**Strain preservation information**

The Saccharomyces cerevisiae AMCC31248 strain (Saccharomyces cerevisiae AMCC31248) provided by the present invention was deposited at the China Center for Type Culture Collection (CCTCC) on December 29, 2021, under the deposit number CCTCC NO: M 20211686, deposited at Wuhan University, Wuhan, China, postal code: 430072; TEL: 027-68754052.

The Saccharomyces cerevisiae AMCC30010 strain (Saccharomyces cerevisiae AMCC30010) used in the present invention was deposited at the China Center for Type Culture Collection (CCTCC) on March 29, 2022, under the deposit number CCTCC NO: M 2022340, deposited at Wuhan University, Wuhan, China, postal code: 430072; TEL: 027-68754052.

The Saccharomyces cerevisiae AMCC32101 strain (Saccharomyces cerevisiae AMCC32101) provided by the present invention was deposited at the China Center for Type Culture Collection (CCTCC) on March 29, 2022, with the deposit number CCTCC NO: M 2022341, deposited at Wuhan University, Wuhan, China, postal code: 430072; TEL: 027-68754052.

**Brief Description of the Drawings**

FIG. 1 shows a colony diagram of the strain Saccharomyces cerevisiae AMCC31248;
FIG. 2 shows a sporulation map of Saccharomyces cerevisiae strain AMCC31248;
FIG. 3 shows the growth curves of parental and new strains in a wort extract medium.

**Detailed Description of the Invention**

According to the present invention, a Saccharomyces cerevisiae AMCC31248 strain provided by the present invention is obtained by a micro hybridization method using a Saccharomyces cerevisiae AMCC30010 strain and a Saccharomyces cerevisiae AMCC32101 strain as parents. Saccharomyces cerevisiae AMCC30010 (Saccharomyces cerevisiae AMCC30010) strain is a Saccharomyces cerevisiae strain bred by Angel Yeast Co. Ltd. which was collected from Yichang City, Hubei Province. The cell morphology of the Saccharomyces cerevisiae strain was observed by light microscopy to be oval, budding, and a single colony growing on a solid plate with a slightly raised central spherical shape, milky white, smooth surface, and neat edge. Morphological observation and molecular biological identification by high-power microscope confirmed that it is a strain of Saccharomyces cerevisiae, which is a food product attribute. It was deposited with the China Center for Type Culture Collection (CCTCC) on March 29, 2022, with the deposit number CCTCC NO: M 2022340 (i.e. CCTCC M 2022340).

Saccharomyces cerevisiae AMCC32101 (Saccharomyces cerevisiae AMCC32101) strain is a Saccharomyces cerevisiae strain bred by Angel Yeast Co. Ltd. which was collected from the city of Ulanqab, Inner Mongolia Autonomous Region. The cell morphology of the Saccharomyces cerevisiae strain was observed by light microscopy to be oval, budding, and a single colony growing on a solid plate with a slightly raised central spherical shape, milky white, smooth surface, and neat edge. Morphological observation and molecular biological identification by high-power microscope confirmed that it is a strain of Saccharomyces cerevisiae, which is a food product attribute. It was deposited with the China Center for Type Culture Collection (CCTCC) on March 29, 2022, with the deposit number CCTCC NO: M 2022341 (i.e. CCTCC M 2022341).

The strain of Saccharomyces cerevisiae AMCC31248 strain provided by the present invention has good fermentation performance in sugar-free dough and can ferment sugar-free dough rapidly.

Some sugar-free bread, soda biscuits, and steamed bread are mainly made by sugar-free dough fermentation. Most of the flour is starch, which is converted into maltose under the action of amylase in flour. Therefore, the maltose utilization ability of yeast determines the rising speed of sugar-free dough. The maltose utilization enzymes of yeast include maltase and maltose permease. The yeast with high maltose utilization ability is called fast-fermenting yeast.

Sugar-tolerant yeast means that it has a higher tolerance to sucrose in sugar-containing doughs, i.e. the growth and fermentation properties in sugar-containing breads are higher than in normal yeasts.

Low sugar tolerant yeasts are used in about 7% sucrose dough systems, and high sugar tolerant yeasts are used in higher sucrose concentration dough systems, up to 25%. Sucrose is generally not directly available to microorganisms, whereas Saccharomyces cerevisiae contains a sucrose hydrolyzing enzyme that degrades sucrose, which acts on the beta -1,2 glycosidic linkages to hydrolyze sucrose to D-glucose and D-fructose, which then enters the glycolytic pathway for use by the yeast, while the glucose and fructose produced by the rapid decomposition of sucrose increase the osmotic pressure around the yeast cells. The yeast cell membrane is a selective semi-permeable biofilm. The activity of yeast cells is affected by the concentration of the external environment. When the cells are in a high osmotic pressure environment, the water content and protoplast in the cells will leak out of the cell membrane to make the cells dehydrated and even die. Therefore, the high osmotic environment that Saccharomyces cerevisiae faces in high-sugar dough has an effect on its growth and fermentation performance. Therefore, the gas production capacity of sugar-free yeast is determined by maltose utilization enzyme activity, and the gas production capacity of sugar-tolerant yeast is determined by sucrase activity.

The reagents and instrument source information used in the examples of the present invention are shown in the following Tables 1 and 2.

Table 1 Reagents information table

| Reagents | Vendor |
| --- | --- |
| Yeast extract powder | Angel yeast |
| Peptone | Angel yeast |
| Glucose | SCR, SINOPHARM |
| Agar | Huixing |
| Potassium acetate | SCR, SINOPHARM |

Table 2 Instrument information sheet

| Instrument | Model | Vendor |
| --- | --- | --- |
| Constant temperature shaker | ZWYR-2102C | Shanghai Zhicheng |
| Biochemical incubator | SPX-150BIII | Tianjin Taiste |
| Bechtop | SKJH-1109 | Shanghai Sukun |
| Clean bench | ME4002E | METTLER TOLEDO |
| pH meter | PB-10 | Sartorius |
| Thermostat water bath | HH-2 | Jiangsu Guohua |
| Centrifuge | DL-5200B | Shanghai Anting |
| Rapid moisture meter | MJ33 | METTLER TOLEDO |
| PCR instrument | C1000 | BIO-RAD |
| Gel imaging system | Gel Doc™ XR⁺ | BIO-RAD |
| Electrophoresis apparatus | EPS-300 | Shanghai Tianneng |
| Light microscope | CX43 | OLYMPUS |
| Fully automatic growth curve analyzer | BioscreenC | OY Growth Curves |
| Yeast micromanipulator | MSM 400 | SINGER |

The formulation of the sporulation medium used in the examples of the present invention was: 1% potassium acetate, 0.1% yeast extract powder, 0.05% glucose, and 2% agar.

In the examples of the present invention, each Saccharomyces cerevisiae strain was activated using a YPD solid medium, the formulation of the YPD solid medium was as follows: 1% yeast extract powder, 2% peptone, 2% glucose, and 2% agar.

In the examples of the present invention, each Saccharomyces cerevisiae strain was cultured in a YPD liquid medium: the formulation of the YPD liquid medium was as follows: 1% yeast extract powder, 2% peptone, and 2% glucose.

**Example 1 Strain construction and identification**

The parent strains Saccharomyces cerevisiae AMCC30010 strain and Saccharomyces cerevisiae AMCC32101 strain were activated and induced to sporulate, respectively. After the enzymolysis of bacterial cells, the single spores were picked using a yeast micromanipulator, and the single spores from two different parents were contacted, and then cultured at 30°C. The spore morphology was observed, and the strain was continued to incubate at 30°C after two single spores were successfully hybridized. This was a first-generation strain. The first-generation strains were further crossed with the Saccharomyces cerevisiae AMCC30010 strain to obtain the second-generation strains, which were then tested for sporulation, and heterozygous strains were selected for subsequent screening.

The growth curve of the new heterozygous strains was determined by the fully automatic growth curve analyzer Bioscreen C, and the heterozygous strains with higher growth efficiency than their parents and ranked first 20 were selected.

A shake flask fermentation test was performed on 20 heterozygous strains with higher growth efficiency than their parents. The net dry weight of the strain and the fermentation activity of fresh yeast in the 0% sugar dough system were used as screening indicators, heterozygous strains with net dry weight of yeast milk reaching 95-105% of either parent and 0% sugar dough fermentation activity reaching 95%-150% of either parent were selected.

Subsequently, the heterozygous strains obtained by the above-mentioned screening step were cultured in a 45 L fermenter system, and the obtained yeast cells were respectively prepared into active dry yeasts, and the fermentation activity of the active dry yeasts in a 0% sugar dough system was determined, and a new strain was selected which has no obvious abnormality during the preparation of the dry yeasts, and the 0% sugar dough fermentation activity of the active dry yeasts can reach 95%-120% of that of any parent.

Finally, the heterozygous strains preferred in the above-mentioned steps were screened for cold osmotic shock resistance. 0% sugar dough containing heterozygous strain active dry yeast was prepared with crushed ice and flour at 0°C by kneading dough, and the leavening time of the dough was determined. The heterozygous strain with the shortest leavening time of the corresponding dough was used as the target strain to screen out the heterozygous strain with cold osmotic shock resistance.

A heterozygous strain named AMCC31248 was obtained by the above-mentioned screening, which had high fermentation activity in 0% sugar dough and good cold osmotic shock resistance. The strain was identified and the identification result was:

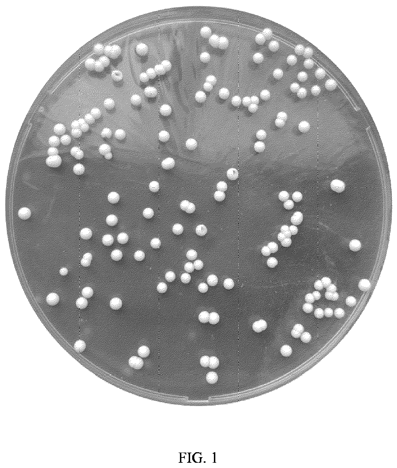

Under the light microscope, it was observed that the cell morphology of the strain was oval, sprouting, and growing. The single colony grown on the solid plate was in a slightly raised central spherical shape, milky white, loose texture, easily lifted by the inoculation loop, smooth surface, drier and neat edge. FIG. 1 shows the colony diagram of heterozygous strain AMCC31248.

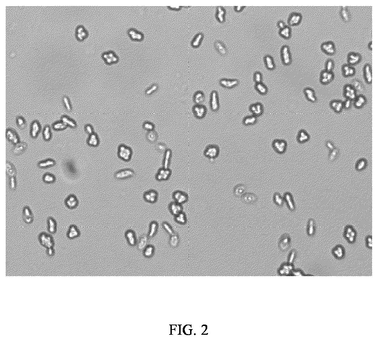

Microscopic examination of sporulation of the heterozygous strain AMCC31248 was shown in FIG. 2, which showed that the heterozygous strain had sporulation ability as indicated by more spores and full morphology in the microscopic field.

The resulting heterozygous strain AMCC31248 was named Saccharomyces cerevisiae AMCC31248 strain (Saccharomyces cerevisiae AMCC31248). This Saccharomyces cerevisiae strain AMCC31248 was deposited at the China Center for Type Culture Collection (CCTCC) on December 29, 2021, with the deposit number CCTCC NO: M 20211686 (i.e. CCTCC M 20211686).

**Example 2 Growth efficiency determination**

The Saccharomyces cerevisiae AMCC31248 strain obtained in Example 1 and parent strains Saccharomyces cerevisiae AMCC30010 and Saccharomyces cerevisiae AMCC32101 were inoculated into wort extract medium (purchased from Hope Bio-Technology Co., Ltd), cultured at 30°C for 48 h, and the $OD_{600}$ value of each strain at different times were determined by high-throughput analysis using the fully automatic growth curve analyzer Bioscreen C. The growth curve was drawn with time (h) as abscissa and the corresponding $OD_{600}$ value as ordinate. The test data were analyzed and the growth efficiency of the strains was calculated according to the following formula.

$$\text{Growth efficiency} = (OD2 - OD1)/(t2 - t1)$$

OD1: $OD_{600}$ value corresponding to strain at t1;
OD2: $OD_{600}$ value corresponding to strain at t2;
t1: the start of the logarithmic growth phase;
t2: the end of the logarithmic growth phase.

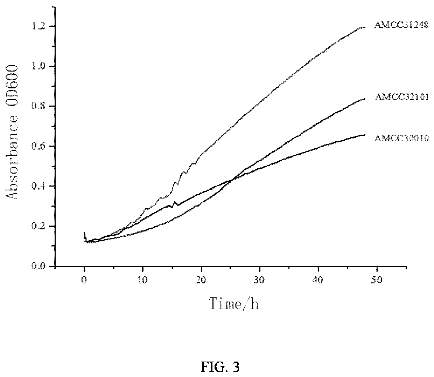

The growth curve of Saccharomyces cerevisiae AMCC31248 is shown in FIG. 3. It can be seen that the strain grows rapidly in the wort extract medium. The growth efficiency of the parent strain and the Saccharomyces cerevisiae AMCC31248 strain is shown in Table 3 below.

Table 3 Growth efficiency data of parent and new strains

| Strain name | Growth efficiency |
|---|---|
| AMCC30010 | 0.013 |
| AMCC32101 | 0.017 |
| AMCC31248 | 0.028 |

As can be seen from above Table 3, the growth efficiency of the obtained Saccharomyces cerevisiae AMCC31248 strain was significantly higher than that of the parent strains Saccharomyces cerevisiae AMCC30010 and Saccharomyces cerevisiae AMCC32101.

**Example 3 Fresh yeast fermentation activity assay**

The Saccharomyces cerevisiae AMCC31248 obtained in Example 1 was inoculated into a shake flask containing a fermentation medium and cultured at 30°C. The precipitate collected after centrifugation, i.e. the yeast milk, was weighed and the water content of the yeast milk was determined. The net dry weight (g/L) of each strain in the shake flask was calculated according to the following formula:

$$\text{Net dry weight (g/L)} = \text{weight of yeast milk x (1 - water content)}$$

According to the 0% sugar dough system shown in Table 4, the dough was prepared to test the fermentation activity of the Saccharomyces cerevisiae AMCC31248 strain, the mass of yeast milk of Saccharomyces cerevisiae AMCC31248 strain and parent strains Saccharomyces cerevisiae AMCC30010 and Saccharomyces cerevisiae AMCC32101 required to be added was calculated and weighed, respectively; according to the dough formulation shown, flour, salt, and water were weighed respectively, and mixed in a dough kneader to prepare the dough; an SJA fermenter was used to directly determine the total volume of carbon dioxide gas produced by yeast fermentation of 280 g dough prepared by the system shown in Table 4 at 30°C for 1 h, i.e. the fermentation activity of the strain, and the results were expressed in milliliters (mL).

Table 4 0% Sugar Dough System

| Raw material | Flour/g | Salt/g | Water/g | Dry yeast/g |
|---|---|---|---|---|
| Addition amount | 280 | 4 | 144 | 2.8 |

The relative percentage of net dry weight in Table 5 was calculated according to the following formula:

Relative percentage of net dry weight (%) = (Net dry weight of heterozygous new strain/Net dry weight of parental strain) * 100%

The relative percentage of dough fermentation activity of the Saccharomyces cerevisiae AMCC31248 strain compared to the parent strain in Table 6 was calculated according to the following formula:

Relative percentage of dough fermentation activity (%) = dough fermentation activity of hybrid new strain/dough fermentation activity of parent strain* 100%

As shown in Table 5, the net dry weight of the Saccharomyces cerevisiae AMCC31248 strain was 95.7% and 109.9% of that of parent strains Saccharomyces cerevisiae AMCC30010 and Saccharomyces cerevisiae AMCC32101, respectively. As shown in Table 6, the 0% sugar dough fermentation activity of the Saccharomyces cerevisiae AMCC31248 strain was superior to both parent strains, with an advantage of about 10%.

Table 5 Net dry weight data of parent and new strains

| Strain name | Net dry weight/(g/L) | Relative percentage % | Relative percentage % |
|---|---|---|---|
| AMCC30010 | 13.37 | 100.0 | 114.8 |
| AMCC32101 | 11.65 | 87.1 | 100.0 |
| AMCC31248 | 12.80 | 95.7 | 109.9 |

Table 6 Fermentation activity data of fresh yeast in 0% sugar dough system

| Strain name | 0% sugar 1 h activity/mL | Relative percentage % | Relative percentage % |
|---|---|---|---|
| AMCC30010 | 1050 | 100.0 | 103.1 |
| AMCC32101 | 1018 | 97.0 | 100.0 |
| AMCC31248 | 1145 | 109.0 | 112.5 |

**Example 4 Active dry yeast fermentation activity assay**

The strain AMCC31248 of Saccharomyces cerevisiae was activated and cultured in a 45 L fermenter. Then the activated dry yeast was isolated, washed, filtered, and dried. Flour, salt, water, and prepared active dry yeast were respectively weighed according to the dough formulation shown in Table 7, and the total amount of carbon dioxide gas produced by fermenting 280 g of the dough prepared by the system shown in Table 7 for 1 h by the active dry yeast was assayed using the SJA method, i.e. the dough fermentation activity of the active dry yeast.

Table 7 0% Sugar dough system

| Raw material | Flour/g | Salt/g | Water/g | Dry yeast/g |
|---|---|---|---|---|
| Addition amount | 280 | 4 | 144 | 2.8 |

Since it is difficult to dry the parent strain AMCC32101 during the preparation of active dry yeast and there is no corresponding fermentation activity data, the dry yeast activity of parent strain AMCC30010 is only used as the control here. There was no obvious abnormality in the preparation of dry yeast of Saccharomyces cerevisiae AMCC31248 strain, and the specific data of 0% sugar dough fermentation activity assay of active dry yeast were shown in Table 8. Among them, the calculation method of the relative percentage of fermentation activity in Table 8 is as follows:

The relative percentage of fermentation activity = (fermentation activity of hybrid new strain/fermentation activity of parent strain AMCC 30010) * 100%

Table 8 Fermentation activity data of active dry yeast in 0% sugar dough system

| Strain name | 0% sugar 1 h activity/mL | Relative percentage of fermentation activity/% |
|---|---|---|
| AMCC30010 | 1016 | 100.0 |
| AMCC31248 | 1133 | 111.5 |

The results showed that compared with the parent Saccharomyces cerevisiae AMCC30010 strain, the Saccharomyces cerevisiae AMCC31248 strain still had an 11.5% advantage in dough fermentation activity under the condition of 0% sugar, indicating that Saccharomyces cerevisiae AMCC31248 strain had certain resistance to drying.

**Example 5 Cold osmotic shock resistance assay of active dry yeast**

According to the dough formulation shown in Table 9, 0°C crushed ice was poured into the dough kneader, active dry yeast was added to mix at low speed, then flour and salt were added, continued mixing and stirring until uniform, and the dough was prepared. After shaping 400 g of dough, it was placed in a leavening box for fermentation, the temperature was controlled to 38 ± 1°C, the humidity was controlled to 85-90%, and the time required for dough fermentation to reach the same set height was recorded, namely, the fermentation time.

Table 9 Cold osmotic shock dough systems

| Raw material | Flour/g | Salt/g | Dry yeast/g | Crushed ice/g |
|---|---|---|---|---|
| Addition amount | 100 | 2 | 1 | 63 |

The fermentation time of the dough corresponding to the strain of Saccharomyces cerevisiae AMCC31248 and the parent strain of Saccharomyces cerevisiae AMCC30010 is shown in Table 10. Among them, the calculation method of the relative percentage of fermentation time in Table 10 is as follows:

The relative percentage of fermentation time = (fermentation time of hybrid new strain/fermentation time of parent strain AMCC30010) * 100%

Table 10 Fermentation time data of active dry yeast in cold osmotic shock dough system

| Strain name | Fermentation time/min | Relative percentage of fermentation time/% |
|---|---|---|
| AMCC30010 | 154.3 | 100.0 |
| AMCC31248 | 108.0 | 70.0 |

The results showed that the fermentation time of the Saccharomyces cerevisiae AMCC31248 strain was only 70.0% of that of the parent Saccharomyces cerevisiae AMCC30010 strain, with an advantage of 30%, indicating that Saccharomyces cerevisiae AMCC31248 strain grew faster and had excellent cold osmotic shock resistance under the same conditions.

While the present invention has been described in connection with what is presently considered to be the most practical and preferred examples, it is to be understood that the present invention is not limited to the disclosed embodiments, but, on the contrary, is intended to cover various modifications and equivalent arrangements included within the spirit and scope of the appended claims.

**Claims**

1. A Saccharomyces cerevisiae strain, **characterized in that** the Saccharomyces cerevisiae strain is: Saccharomyces cerevisiae AMCC31248 strain (Saccharomyces cerevisiae AMCC31248) deposited at the China Center for Type Culture Collection (CCTCC) with the deposit number CCTCC NO: M 20211686.

2. A fermentative preparation method for a Saccharomyces cerevisiae microbial agent, **characterized by** comprising the following steps: culturing the Saccharomyces cerevisiae strain according to claim 1.

3. The preparation method according to claim 2, **characterized in that** the preparation method comprises the following steps:

   (1) amplifying and culturing the Saccharomyces cerevisiae strain according to claim 1;
   (2) adding the product obtained in step (1) to a liquid medium, and fermenting and culturing at 26-32°C.
   4. A microbial agent, **characterized by** comprising the Saccharomyces cerevisiae AMCC31248 strain (Saccharomyces cerevisiae AMCC31248) according to claim 1.
   5. The microbial agent according to claim 4, **characterized in that** the microbial agent is obtained by the fermentative preparation method according to claim 2 or 3.
   6. Use of the Saccharomyces cerevisiae strain according to claim 1 or the microbial agent according to claim 4 or 5 for fermentation.
   7. Use of the Saccharomyces cerevisiae strain according to claim 1 or the microbial agent according to claim 4 or 5 in a dough.
   8. A dough, **characterized by** comprising the Saccharomyces cerevisiae strain according to claim 1 or the microbial agent according to claim 4 or 5.

9. The dough according to claim 8, **characterized in that** the dough comprises flour and the Saccharomyces cerevisiae strain in a mass ratio of 100:0.5-5.

10. A preparation method for the dough according to claim 8 or 9, **characterized in that** the preparation method comprises the following steps: kneading a dough with water at 0-35°C.

11. A flour product, obtained by the preparation method for the dough according to claim 8 or 9.

12. The flour product according to claim 11, being a steamed bread, a steamed stuffed bun, a bread, a biscuit, a noodle, and a pan-fried dumpling.

FIG. 1

FIG. 2

FIG. 3

| INTERNATIONAL SEARCH REPORT | International application No. |
| --- | --- |
| | **PCT/CN2023/079577** |

**A.    CLASSIFICATION OF SUBJECT MATTER**

C12N1/18(2006.01)i;   A21D8/04(2006.01)i;   C12R1/865(2006.01)n

According to International Patent Classification (IPC) or to both national classification and IPC

**B.    FIELDS SEARCHED**

Minimum documentation searched (classification system followed by classification symbols)

IPC:  C12N;  A21D;  C12R

Documentation searched other than minimum documentation to the extent that such documents are included in the fields searched

Electronic data base consulted during the international search (name of data base and, where practicable, search terms used)

CJFD, CNABS, CNTXT, DWPI, ENTXT, ENTXTC, VEN, WPABS, WPABSC, CNKI, PUBMED: 耐冷, 耐冷渗透, 渗透休克, 酿酒酵母, AMCC30010, AMCC31248, Saccharomyces cerevisiae, cold resist+, cold shock, dough develop+

**C.    DOCUMENTS CONSIDERED TO BE RELEVANT**

| Category* | Citation of document, with indication, where appropriate, of the relevant passages | Relevant to claim No. |
| --- | --- | --- |
| A | CN 113355251 A (ANGEL YEAST CO., LTD.) 07 September 2021 (2021-09-07)<br>    see claims 1-10, and description, embodiment | 1-12 |
| A | JP H07213277 A (NIPPON BEET SUGAR MANUFACTURING CO., LTD.; NATIONAL TAX ADMINISTRATION AGENCY;) 15 August 1995 (1995-08-15)<br>    see description, embodiments 5-6 | 1-12 |
| A | CN 103275881 A (TIANJIN UNIVERSITY OF SCIENCE AND TECHNOLOGY) 04 September 2013 (2013-09-04)<br>    see claims 1-4 | 1-12 |
| A | US 2019119763 A1 (NEXTFERM TECHNOLOGY LTD.) 25 April 2019 (2019-04-25)<br>    see abstract | 1-12 |
| A | WO 2012128186 A1 (KANEKA CORP.; IZUMI TAKUYA; TAKATA TAKAHIRO; TAKATA HAYATO;) 27 September 2012 (2012-09-27)<br>    see abstract | 1-12 |

☐ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **24 June 2023** | **06 July 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2023/079577**

| Patent document cited in search report | | | Publication date (day/month/year) | Patent family member(s) | | | Publication date (day/month/year) |
|---|---|---|---|---|---|---|---|
| CN | 113355251 | A | 07 September 2021 | None | | | |
| JP | H07213277 | A | 15 August 1995 | JP | 2943041 | B2 | 30 August 1999 |
| CN | 103275881 | A | 04 September 2013 | None | | | |
| US | 2019119763 | A1 | 25 April 2019 | EP | 3443127 | A1 | 20 February 2019 |
| | | | | US | 11597908 | B2 | 07 March 2023 |
| | | | | KR | 20180133485 | A | 14 December 2018 |
| | | | | KR | 102288790 | B1 | 11 August 2021 |
| | | | | RU | 2018139496 | A | 12 May 2020 |
| | | | | RU | 2018139496 | A3 | 23 September 2020 |
| | | | | RU | 2756307 | C2 | 29 September 2021 |
| | | | | IL | 262248 | A | 29 November 2018 |
| | | | | IL | 262248 | B | 01 June 2022 |
| | | | | JP | 2019511241 | A | 25 April 2019 |
| | | | | BR | 112018071207 | A2 | 12 February 2019 |
| | | | | CA | 3020653 | A1 | 19 October 2017 |
| | | | | WO | 2017178879 | A1 | 19 October 2017 |
| | | | | MX | 2018012582 | A | 04 July 2019 |
| WO | 2012128186 | A1 | 27 September 2012 | JPWO | 2012128186 | A1 | 24 July 2014 |
| | | | | JP | 5907161 | B2 | 20 April 2016 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- NO M20211686